# EUROPÄISCHE PATENTSCHRIFT

(11) **EP 1 487 468 B1**
(45) Veröffentlichungstag und Bekanntmachung des Hinweises auf die Patenterteilung: **31.05.2006**
(21) Anmeldenummer: 03712092.0
(22) Anmeldetag: 25.03.2003
(51) Int. Cl.: A61K 36/15

(54) **PFLANZENEXTRAKTE UND DEREN ANWENDUNG**
PLANT EXTRACTS AND THE USE THEREOF
EXTRAITS DE PLANTES ET LEUR UTILISATION

(30) Priorität: 26.03.2002 DE 10213571
(43) Veröffentlichungstag der Anmeldung: 22.12.2004
(73) Patentinhaber: Lichtwer Healthcare GmbH & Co. KG, 13435 Berlin (DE)
(72) Erfinder: PLOCH, Michael, 10965 Berlin (DE); MURCK, Harald, 10435 Berlin (DE); HAFFNER, Thomas, 13187 Berlin (DE)
(74) Vertreter: HOFFMANN EITLE
(86) Internationale Anmeldenummer: PCT/EP2003/003097
(87) Internationale Veröffentlichungsnummer: WO 2003/080085

(56) Entgegenhaltungen:
- EP-A- 0 599 307
- EP-A- 1 034 782
- WO-A-00/02455
- WO-A-00/04912
- WO-A-99/40905
- WO-A-99/66914
- DE-A- 3 338 995
- US-A- 6 083 932

## Beschreibung

Die vorliegende Erfindung betrifft eine neue Verwendung von Pflanzen und Pflanzenteilen zur Behandlung von Schizophrenie. Insbesondere betrifft die vorliegende Erfindung die Verwendung von Johanniskraut, Gingko, Safran und Ginseng zur Behandlung der Schizophrenie und der dabei auftretenden Negativsymptomatik.

### Stand der Technik

Der Begriff der Schizophrenie stammt von Bleuler (1911) (G. Huber "Psychiatrie", Schattauer Verlag, 5. Auflage, 1994), der das moderne Krankheitskonzept geprägt hat und bezeichnet den Zerfall von Wollen, Fühlen und Handeln eines Patienten, der an dieser Erkrankung leidet. In der akuten Phase herrschen sogenannte Positivsymptome vor, wie 1. Wahrnehmungsstörungen, etwa akustische Halluzinationen von Stimmen, die kommentierenden oder imperativen Charakter haben können, 2. Wahn, etwa der Verfolgungswahn, 3. Denkstörungen, wie etwa Gedankenabreissen bis hin zu völlig zerfahrenem Denken. Diese Störungen müssen länger als 4 Wochen anhalten um die Diagnose Schizophrenie stellen zu können.

Eine vollständige Wiederherstellung der Gesundheit kann auch durch die heutige Medikation nur in ca. 1/3 der Patienten erreicht werden. Der noch vor Bleuler von Kraepelin, dem Vater der modernen psychiatrischen Klassifikation, eingeführte Begriff der "Dementia praecox" für diese Gruppe von Patienten, sieht in dem "vorzeitig" einsetzenden Abbau von kognitiven Fähigkeiten das Kernsymptom. Kraepelin sah in dieser Diagnose, anders als Bleuler, die schlechte Prognose als entscheidend für diese Patienten an. Damit hatten für ihn die sogenannten Negativ- oder Defizitsymptome die größere Bedeutung. Negativ- oder Defizitsymptome sind kognitive Beeinträchtigungen, Apathie, flacher Affekt, Initiativemangel, Sprachverarmung, aber auch verminderte soziale Fähigkeiten und Vernachlässigung der eigenen Körperpflege. Insbesondere diese Symptomatik führt zu beträchtlichen Folgekosten. Die Schizophrenie ist eine psychiatrische Erkrankung, die ca. 1% der Bevölkerung im Verlauf ihres Lebens betrifft. 80% der Betroffenen haben die Erstmanifestation vor dem 40. Lebensjahr. Die jährlichen Kosten aufgrund eines Verlustes von Produktivität und der entstehenden Behandlungskosten betrug in den USA zu Beginn der neunziger Jahre ca. 30-50 Milliarden Dollar jährlich. Für den Einzelnen bedeutet die Erkrankung eine um 20% niedrigere Lebenserwartung, insbesondere 10 % aller Erkrankten begehen Suizid.

Die Schizophrenie ist von der Depression, einer Indikation, für die z.B. Johanniskraut als ein Beispiel für einen Pflanzenextrakt zugelassen ist, durch ihre Symptomatik abgegrenzt, wobei aber auch Übergangsformen und Mischzustände möglich sind. Die allgemeinen Kriterien zur Diagnose einer Schizophrenie sind nach dem international gültigen Klassifikationsschema ICD 10 (Internationale Klassifikation psychischer Störungen, 10 Auflage, herausgegeben von der Weltgesundheitsorganisation) definiert unter den Ziffern F20.0 bis F20.3: Für diese gilt, das die Diagnose zu stellen ist dann,

wenn mindestens 1 Symptom der Symptomgruppe 1 - 4, nämlich: 1 Gedankenlautwerden, Gedankeneingebung, Gedankenentzug, und Gedankenausbreitung; 2. Kontrollwahn, Beeinflussungswahn, Gefühl des Gemachten, Wahnwahrnehmung: 3. kommentierende oder dialogische Stimmen und 4. unrealistischer und kulturell unangemessener Wahn eindeutig nachweisbar ist oder mindestens 2 Symptome der Gruppe 5-8, nämlich 5. anhaltende Halluzinationen jeder Sinnesmodalität; 6. Gedankenabreißen oder Gedankeninterferenz mit konsekutiver Zerfahrenheit, Danebenreden oder Neologismen; 7. katatone Symptome; und 8. negative Symptome wie Affektverflachung, Sprachverarmung und Apathie fast vollständig während eines Monats oder länger nachweisbar sind, kann eine Schizophrenie diagnostiziert werden.

Durch diese Kriterien werden die akuten Schizophrenieformen paranoide Schizophrenie (F 20.0), hebephrene Schizophrenie (F 20.1), katatone Schizophrenie (F 20.2) und undifferenzierte Schizophrenie (F 20.3) festgelegt. Die genauere Differenzierung dieser Formen ist in diesem Zusammenhang unerheblich.

Die Patienten leiden dabei unter massiven Ängsten. Häufig herrscht bei ihnen das Gefühl vor, Ferngesteuert zu sein und auf das eigenen Wollen und Fühlen keinen Einfluss mehr zu haben. Auffällig für den Außenstehenden ist die Reaktion der Betroffenen auf ihre.akustischen Halluzinationen, insbesondere auf die Stimmen, denen die Patienten teilweise antworten, die aber auch zu nicht vorhersehbaren Handlungen der Patienten führen können. Dabei sind von besonderer Relevanz Handlungen, die zum Tod des Patienten führen können, etwa wenn sei aus dem Fenster springen, weil sie den Befehl dazu von einer Stimme erhalten haben. Die akute Schizophrenie mit den dabei auftretenden sogenannten Positivsymptomatik ist die klassische Geisteskrankheit und damit abzugrenzen zur Depression, der klassischen Gemütskrankheit.

Negativ- oder Defizitsymptome sind bereits oben angesprochen worden und sind etwa kognitive Beeinträchtigungen, Apathie, flacher Affekt, Initiativemangel, Sprachverarmung, aber auch verminderte soziale Fähigkeiten und Vernachlässigung der eigenen Körperpflege. Diese Symptome treten insbesondere beim schizophrenen Residuum (ICD 10: F 20.5) oder der Schizophrenia simplex (ICD 10, F20.6) auf, die abgegrenzt werden von den oben genannten Schizophrenieformen 20.0 - 20.3.

Ein Zustand einer akuten Schizophrenie mit der dabei vorherrschenden Positivsymptomatik muss davor gegangen sein, um ein schizophrenes Residuum (ICD-10 F20.5), diagnostizieren zu können, hingegen ist diese Symptomatik für die Diagnose einer Schizophrenia Simplex explizit ausgeschlossen. Bei einer Depression, die klassischerweise episodenhaft verläuft, die aber auch chronifizieren kann, liegt ebenfalls keine Vorgeschichte einer akuten Schizophrenie mit den oben angegebenen Kriterien vor.

Als weitere Merkmale des schizophrenen Residuums sind definiert:
Auftreten von mindestens 4 der folgenden "negativen Symptome" während der vergangenen zwölf Monate:
   1. psychomotorische Verlangsamung oder verminderte Aktivität,
   2. deutliche Affektverflachung,
   3. Passivität und Initiativemangel,
   4. Verarmung hinsichtlich Menge oder Inhalt des Gesprochenen
   5. geringe nonverbale Kommunikation, deutlich an Mimik, Blickkontakt, an Stimmmodulation und Körperhaltung,
   6. verminderte soziale Leistungsfähigkeit und Vernachlässigung der Körperpflege

Diese Patienten erleiden sehr häufig einen sozialen Abstieg und sind daher nicht selten im Obdachlosenmilieu zu finden.

Gleiches gilt für die Patienten mit Schizophrenia simples. Nach ICD 10 sind die diagnostischen Kriterien:

Schleichende Progredienz aller drei folgenden Merkmale über einen Zeitraum von mindestens einem Jahr:
1. deutliche und anhaltende Veränderungen in einigen früheren Perönlichkeitsmerkmalen, was sich in einem Antriebs- und Interesseverlust äußert, sowie in nutz- und ziellosem Verhalten, in Selbstversunkenheit und sozialem Rückzug.
2. allmähliches Auftreten und Verstärtkung non "negativen" Symptomen wie Apathie, Sprachverarmung, verminderte Aktivität, deutliche Affektverflachung, Passivität, Inititativemangel und verminderte nonverbale Kommunikation (Mimik, Blickkontakt, Stimmodulation oder Körperhaltung)
3. deutliche Abnahme der schulischen und beruflichen Leistungsfähfigkeit.

Die Symptomatik der beiden Störungen der Schizophrenia simplex und des schizophrenen Residuums gleichen sich in weiten Teilen. Von der Depression lässt sich diese Symptomatik abgrenzen insbesondere durch das Fehlen einer im Vordergrund stehenden niedergedrückten Stimmung im Gegensatz zur Affektverarmung hier sowie der unterschiedliche zeitliche Verlauf der Störungen.

Für die Therapie von Schizophrenie wurden bisher folgende Konzepte entwickelt und eingesetzt.

Nach der Einführung synthetischer Neuroleptika in den 50er Jahren des 20. Jahrhunderts standen die Positivsymptome im Vordergrund des Interesses, da diese durch die neuen Substanzen gut behandelt werden konnten. Das erste synthetisierte Neuroleptikum Chlorpromazin ist ein Phenothiazin-Derivat und Vorläufer einer ganzen Gruppe von ähnlichen Substanzen. Daneben wurden eine andere Substanzklasse, die der Butyrophenone wie z.B. Haloperidol für diese Indikation entwickelt. Gemeinsam ist allen diesen Substanzen eine blockierende Eigenschaft an bestimmten Dopamin-Rezeptoren. Sie werden als "klassische Neuroleptika" zusammengefasst.

Das erste synthetische Antidepressivum Imipramin wurde einige Jahre später entwickelt. Dabei handelt es sich um ein sogenanntes trizyklisches Antidepressivum, dessen Wirkmechanismus in der Hemmung der Wiederaufnahme bestimmter Neurotransmitter, nämlich Noradrenalin und Serotonin, liegt. Die größte Anzahl der derzeit verwendeten synthetischen Antidepressiva basiert auf dieser Eigenschaft der Substanzen. In Abgrenzung zu den Neuroleptika sind Antidepressiva unwirksam bei der Behandlung der akuten Schizophrenie.

Erst mit der Einführung des ersten sogenannten atypischen Neuroleptikums Clozapin Ende der 70er Jahre kamen die Defizitsymptome wieder in den Focus des Interesses, da Clozapin auch auf diese einen deutlichen Einfluss hat. Dies ist insbesondere daher der Fall, weil neben Clozapin, das lange Zeit das einzige verfügbare atypische Neuroleptikum war, mittlerweile eine ganze Palette davon existiert, wie in einer Übersichtsarbeit von Möller (Möller H-J, (2000): Nervenarzt 71:345-353) dargestellt. In dieser Übersichtsarbeit wird zur Frage der Negativsymptomatik ausgeführt: "eine klinisch ganz bedeutsame Frage, weil die chronische Negativsymptomatik im wesentlichen der Verlauf schizophrener Erkrankungen bestimmt". Auch zentrale Werbeaussagen der neuen Substanzen zielen auf die Effizienz bei Negativsymptomatik hin. Es bestehen jedoch einige Unterschiede zwischen diesen neuen Substanzen sowohl im Hinblick auf ihr Wirk- wie auch Nebenwirkungsprofil. Clozapin-Einnahme ist mit dem Risiko einer Agranulozytose, d.h. eines Verschwindens bestimmter weißer Blutkörperchen, verbunden und daher von Seiten der Vertreiberfirma mit deutlichen Auflagen versehen. Sowohl Clozapin als auch Olanzapin führen zu einer ausgeprägten Gewichtszunahme, in geringerem Maße gilt dies auch für Risperidon. Möller schreibt dazu: "Diese z.T. erhebliche Gewichtszunahme mit ihren u.a. medizinischen Konsequenzen wird wahrscheinlich in Zukunft eine zentrale Stellung in der Nebenwirkungsproblematik der Neuroleptika haben".

Aufgabe der vorliegenden Erfindung ist es somit ein Medikament bereitzustellen, das die oben genannten Nachteile nicht aufweist und die Schizophrenie, insbesondere die Negativsymptomatik bei der Schizophrenie therapiert.

### Kurze Zusammenfassung der Erfindung

Die vorliegende Erfindung betrifft die Verwendung von Pflanzen und Pflanzenextrakten zur Behandlung von Schizophrenie. Insbesondere betrifft die vorliegende Erfindung die Verwendung von Johanniskraut, Gingko, Safran und Ginseng zur Behandlung von Schizophrenie. Dafür sind besonders Pflanzenextrakte der oben genannten frischen und getrockneten Pflanzen geeignet.

Bei diesen Pflanzenextrakten handelt es sich bevorzugt um alkoholische oder alkohol-wässrige Auszüge.

Die erfindungsgemäße Verwendung der Stoffe erfolgt in den üblichen pharmazeutischen Darreichungsformen, wie flüssigen, halbfesten und festen Formen.

Weiterhin betrifft die Erfindung die kombinierte Verwendung der o.g. Stoffe mit herkömmlichen Therapeutika bei der Behandlung von Schizophrenie. D.h. die Stoffe können als Kombinationspräparat mit üblichen Medikamenten zur Behandlung von Schizophrenie zur gleichzeitigen, getrennten oder zeitlich abgestuften Anwendung bei Schizophrenie verwendet werden.

Schließlich richtet sich die vorliegende Erfindung auf Verfahren zur Behandlung von Individuen, die an Schizophrenie leiden.

### Ausführliche Beschreibung der Erfindung

Die neue Indikation für Johanniskraut, Gingko, Safran und Ginseng ist die Behandlung der Schizophrenie und besonders die Behandlung der Negativsymptomatik insbesondere bei Patienten mit chronischer Schizophrenie. Die Medikation kann dabei als Add-On Therapie zu einer Therapie mit einem üblichen Psychopharmaka zur Behandlung der Schizophrenie , wie einem Neuroleptikum vorgesehen werden, d.h. unter anderem als Kombinationspräparat mit herkömmlichen Neuroleptika oder anderen zur Behandlung der Schizophrenie eingesetzten Psychopharmaka.

Vorteilhaft ist dabei gemäß einer Ausführungsform die zusätzliche Gabe von z.B. Johanniskraut-Extrakt zu einer Basis-Medikation eines klassischen Neuroleptikums, insbesondere bei Patienten, die über einen langen Zeitraum darauf eingestellt sind und insgesamt psychopathologisch stabil sind.

Als klassische Neuroleptika können insbesondere erwähnt werden Substanzen wie Haloperidol, Benperidol, Chlorprotixen, Flupentixol, Fluphenazin, Perazin, Perphenazin, Thioridazin. Möglich ist aber auch die Add-on Therapie bei einer Basis-Therapie mit atypischen Neurolpetika, wie etwa Clozapin, Olanzapin, Seroquel, Sertindol, aber auch alle übrigen zur Behandlung der Schizophrenie geeigneten Psychopharmaka.

Das heißt, erfindungsgemäß wird gemäß einer Ausführungsform ein Kombinationspräparat, enthaltend Johanniskraut, Gingko, Safran und/oder Ginseng sowie als weitere Komponente ein herkömmliches Psychopharmaka gegen Schizophrenie zur gleichzeitigen, getrennten oder zeitlich abgestuften Anwendung bei der Behandlung von Schizophrenie bereitgestellt.

Dieses Kombinationspräparat oder eine einfache Kombination von den beanspruchten Pflanzenpräparaten mit Psychopharmaka zur Behandlung der Schizophrenie, wie etwa Neuroleptika, insbesondere aus der Gruppe der klassischen Neuroleptika wie Haloperidol, Benperidol, Chlorprotixen, Flupentixol, Fluphenazin, Perazin, Perphenazin, Thioridazin können Verwendung bei der Add-on-Therapie finden. Möglich ist aber auch die Add-on Therapie bei einer Basis-Therapie mit atypischen Neurolpetika, wie etwa Clozapin, Olanzapin, Seroquel, Sertindol, aber auch alle übrigen zur Behandlung der Schizophrenie geeigneten Substanzen.

Nicht erfolgen sollte die Gabe von z.B. Johanniskraut-Extrakt bei bestehenden floriden psychotischen Symptomen wie Wahrnehmungsstörungen oder Wahn.

Johanniskraut (Hypericum perforatum) kann in frischer oder getrockneter Form vorliegen. Zur direkten Anwendung sowie zur Extraktgewinnung können alle Teile der Pflanze in Abhängigkeit des Wachstumsstadiums, vorzugsweise die oberirdischen Teile wie z.B. Blüten, Blätter, Stengel und Samenstände verwendet werden. Typische Inhaltsstoffe sind Hypericine, Hyperforine, Flavonoide und Biaflavone.
Ginkgo (Ginkgo biloba) kann in frischer, vorzugsweise getrockneter Form vorliegen. Zur direkten Anwendung sowie zur Extraktgewinnung werden bevorzugt Blätter verwendet. Typische Inhaltsstoffe sind die Flavonglycoside und Terpenlactone, z.B. Bilobalid, Ginkgolide.
Safran (Crocus sativus) kann in frischer oder getrockneter Form vorliegen. Zur direkten Anwendung sowie zur Extraktgewinnung können alle Teile der Pflanze in Abhängigkeit des Wachstumsstadiums, vorzugsweise die oberirdischen Teile, besonders bevorzugt die Blütennarben verwendet werden. Typische Inhaltsstoffe sind α-, β-Pinen, 1,8-Cineol, das Carotinoid Crocin, Picrocrocin sowie dessen Abbauprodukt Safranal.
Ginseng (Panax ginseng) kann in frischer oder getrockneter Form vorliegen. Zur direkten Anwendung sowie zur Extraktgewinnung können alle Teile der Pflanze in Abhängigkeit des Wachstumsstadiums, vorzugsweise die Wurzeln verwendet werden. Typische Inhaltsstoffe sind Triterpensaponine (Ginsenoside), Sesquiterpene und Polyacetylene.

Das Auszugsmittel bei den erfindungsgemäßen ein- oder mehrstufigen Verfahren ist bevorzugt ein nicht-wässriges Auszugsmittel, wie ein organisches Lösungsmittel oder ein wässrig-alkoholisches Auszugsmittel. Beispielhaft seien hier Alkohole, Ketone, Ester, Ether, Aromaten, halogenierte Verbindungen, Alkane, Alkene usw. genannt, in reiner Form oder als Gemische mit Wasser. Insbesondere sind aliphatische Alkohole, Ketone und Karbonsäureester geeignet. Diese Lösungsmittel können alleine oder als Mischung der obigen Verbindungen verwendet werden. Über- und unterkritische Gase wie z.B. Kohlendioxid (CO₂) und Ethan sind ebenfalls verwendbare Auszugsmittel, auch in Mischung mit oben genannten Verbindungen. Beispielhaft seien reines überkritisches Kohlendioxid oder gemischt mit weiteren Lösungsmitteln wie z.B. Methanol genannt.

Der verwendete Extrakt kann ein alkoholischer, alkoholischwässriger Auszug mit primären, sekundären und tertiären Alkoholen der Reihe C1-C5, vorzugsweise Methanol und Ethanol sein, in der Zusammensetzung von Alkohol/Wasser zwischen 100/0 bis 30/70, vorzugsweise 80/20 bis 50/50.

In einer besonders bevorzugten Ausführungsform wird als Auszugsmittel für Johanniskraut 80% Methanol (20% Wasser), für Gingko 60% Aceton (40% Wasser) auch in Verbindung mit Umfällungsschritten und nachgeschalteten Extraktionsschritten z.B. mit Ketonen, Butanol und Toluol, für Safran > 90% Ethanol (< 10% Wasser), und für Ginseng 30% Ethanol (70 % Wasser) verwendet.

Die Extrakte werden aus dem frischen oder getrockneten Ausgangsmaterial mit Hilfe unbewegter Extraktionsverfahren z.B. Mazeration und bewegter Extraktionsverfahren z.B. Perkolation, Ultraturrax-, Ultraschallextraktion gewonnen werden (Flüssig-Flüssig-Verfahren) gewonnen. Sie liegen direkt als Flüssigextrakte oder aufkonzentrierte Dickextrakte (Spissum) oder in Form ihrer Trockenextrakte vor. Bei Johanniskraut, Ginkgo, Ginseng bevorzugt sind Trockenextrakte bevorzugt, bei Safran die Flüssig-, bzw. Dickextrakte. Johanniskrautextrakte enthalten bevorzugt Hypericine, Hyperforine und Flavonoide, besonders bevorzugt in folgenden Konzentrationen:
0,01 - 2 % Hypericine, 0,01 - 30 % Hyperforine, 2 - 35 % Flavonoide, vorzugsweise 0,10 - 0,40 % Hypericine und 1 - 6 % Hyperforine aufweist.
Extrakte aus Ginkgo biloba Blättern enthalten Flavonglycoside und Terpenlactone, besonders bevorzugt in folgenden Konzentrationen: 20 - 30 Gew. % Flavonglycoside, zusammen 2 - 8 Gew. % der Ginkgolide, besonders bevorzugt 23 - 27 % Flavonglycoside und 5 - 7 Gew. % Ginkgolide aufweist. Extrakte aus Safran enthalten u.a. α-, β-Pinen, 1,8-Cineol, Crocin, Picrocrocin sowie ggf. dessen Abbauprodukt Safranal, besonders bevorzugt in folgenden Konzentrationen: 5 - 10% Pinene und Cineol, 4 - 10% Picrocrocin und/oder 2 - 6% Safranal. Extrakte aus Ginsengwurzeln enthalten u.a. Triterpensaponine (Ginsenoside/Ginsenoide), Sesquiterpene und Polyacetylene, besonders bevorzugt in folgenden Konzentrationen: 3 - 9% Ginsenoide.

Die beschriebenen Stoffe aus den oben genannten Pflanzen können in üblichen festen, halbfesten und flüssigen pharmazeutischen und sonstigen Darreichungsformen verarbeitet und appliziert werden, wie z.B. in Pulvern, Lösungen, Suspensionen, Tabletten, Filmtabletten, Dragees, Kapseln, Brausetabletten, Brausegranulat, Kau-, Lutschtabletten, Suppositorien, Cremes, Salben, Gelen. Dabei können für die jeweilige Darreichungsform übliche Hilfsstoffe verwendet werden, wie z.B. Cellulosen, Siliciumdioxide, Lactose, natürliche und synthetische Polymere, Salze, Farbstoffe, Aromastoffe, Fette, Öle, Tenside, Wasser und Alkohole.

Die Tagesdosis für Johanniskraut Extrakt soll dabei in einem Bereich von 300 mg Extrakt bis 2700 mg Extrakt liegen, bevorzugt bei 1500 mg Extrakt aufgeteilt in zwei Einzeldosen.

Für Gingko-Ektrakt, Saffran-Extrakt und Ginseng-Extrakt ist eine Dosisspanne von 50 mg Extrakt bis 1000 mg Extrakt zu bevorzugen. Natürlich spielen bei der Bestimmung der Dosierung der Zustand des Individuums eine Rolle, wie Alter, Gewicht, etc.

Weiterhin wird erfindungsgemäß ein Behandlungsverfahren der Schizophrenie bereitgestellt. Dieses Verfahren umfasst die Verabreichung von Hypericum perforatum (Johanniskraut) und/oder Ginkgo biloba (Ginkgo) und/oder Crocus sativus (Safran) und/oder Panax ginseng (Ginseng) an Individuen, die an Schizophrenie leiden.

Insbesondere werden dabei die Pflanze, Pflanzenteile, getrocknete Pflanze oder Pflanzenteile, Extrakte, Extraktfraktionen, Reinstoffe und deren Derivate und Salze aus der Pflanze zur Verabreichung an Individuen zur Behandlung der Schizophrenie verwendet.

Besonders vorteilhaft handelt es sich dabei um die oben beschriebenen Extrakte der einzelnen Pflanzen. Diese können allein oder in Kombination im Behandlungsverfahren verabreicht werden.

Das erfindungsgemäße Verfahren zur Behandlung schließt auch die gleichzeitige, getrennte oder zeitlich abgestufte Gabe der oben genannten Wirkstoffe mit einen herkömmlichen Psychopharmaka gegen Schizophrenie zur der Behandlung von Schizophrenie ein.

Besonders vorteilhaft handelt es sich bei dem Behandlungsverfahren um eine Add-on Therapie der oben genannten Wirkstoffe in Kombination mit Psychopharmaka zur Behandlung der Schizophrenie, insbesondere Neuroleptika, insbesondere aus der Gruppe der klassischen Neuroleptika wie Haloperidol, Benperidol, Chlorprotixen, Flupentixol, Fluphenazin, Perazin, Perphenazin, Thioridazin, atypischen Neuroleptika, insbesondere Clozapin, Olanzapin, Seroquel, Sertindol, sowie andere zur Behandlung der Schizophrenie geeignete Psychopharmaka.

### Beispiel

Ein gut etabliertes Modell der kognitiven Störungen bei chronisch schizophrenen Patienten ist die Induktion von Denkstörungen durch Verabreichung des Anästhetikums Ketamin {Duncan G. et al (2000) J Pharmacol Exp Ther 293:8-14, Krystal JH, et al (2000):Biol Psychiatry 47:137-143}, die durch atypische Neuroleptika wie Olanzapin und Clozapin vermindert werden {Duncan, ibid}. JOHANNISKRAUT-EXTRAKT hat in einer tierexperimentellen Untersuchung einen Antagonismus des Effekts von Ketamin auf die Induktion von Schlaf gezeigt (Butterweck V, et al (1997): Pharmacopsychiatry 30 Suppl 2:117-124).

Im Folgenden wurde der Ketamin-antagonistischen Effekt von Hypericumextrakt als ein Parameter untersucht, der eine mögliche Wirksamkeit von Johanniskraut auf die Negativsymptomatik von Patienten mit chronischer Schizophrenie aufzeigt. Dazu wurde untersucht, ob die subchronische Gabe von 2 x 750 mg Johanniskraut-Extrakt (Jarsin® 750 mg, Lichtwer Pharma AG, Berlin, Deutschland) nach 7 Tagen oraler Einnahme von je 1 Tablette morgens und abends einen Einfluss auf durch Ketamin hervorgerufene Veränderungen bewirkt. Diese Studie war in doppelblind randomisiertem Design angelegt und wurde an 16 gesunden Probanden durchgeführt. Dabei wurde ein sogenanntes "Cross-Over-Design" verwendet: die Hälfte der Probanden erhielt über eine Woche Placebo, es folgte eine Woche ohne Medikation, darauf eine Woche Gabe von Johanniskraut-Extrakt. Die andere Hälfte erhielt in der ersten Woche Johanniskraut-Extrakt und in der letzten Placebo. Die Probanden wurden den Gruppen zufällig zugeteilt.

Als Parameter wurde die Veränderung elektrophysiologisch zu bestimmender Messwerte durch die Gabe von Ketamin gewählt, außerdem wurde die Veränderung der kognitiven Fähigkeiten in einem speziellen Test untersucht.

Im Einzelnen wurde die Veränderung der Differenz der Peaks N1-P2 des akustisch evozierten Potentials (AEP) als primärer Zielparameter herangezogen. Dabei wurde ein Stimulus von 100 ms Dauer mit einer Frequenz von 1 kHz in zufälliger Weise über einen Kopfhörer einem Ohr zugespielt. Die Lautstärke des Tones war konstant bei 90 dBA, die Abstände zwischen den Tönen variierten zwischen 4 und 8 s. Da AEPs keiner Habituation unterliegen, sind sie für eine Untersuchung im Cross-Over-Design gut geeignet.

Als sekundäre Zielparameter wurde die Veränderung weiterer elektrophysiologisch zu bestimmender Parameter gewählt, nämlich die der Latenz, die der Winkelgeschwindigkeit, die der Zeit bis zur Fixierung, die der Reaktionszeit bis zu einer erfolgten Auswahl und die der Anzahl der richtigen Reaktionen beim Okkulodynamischen Test (ODT). Der ODT liefert Parameter der zentralnervösen Leistungsfähigkeit, die nicht durch veränderte Motivation oder Lernen beeinflusst werden. Sie sind daher ebenfalls gut für ein Cross-Over-Design geeignet. Beim ODT werden Licht-Signale in definierten horizontalen Positionen der visuellen Achse in zufälliger Reihenfolge präsentiert. Dabei werden insgesamt 900 Signale innerhalb von 15 min. benutzt. Mittels eines Elektrooculograms (EOG) lassen sich die Augenbewegungen messen und folgende Parameter gewinnen:
1. Mittlere Latenz zwischen dem Erscheinen des Licht-Signal und dem Beginn der Augenbewegung
2. Mittlere Winkelgeschwindigkeit der Augenbewegung
3. Zeit bis zur Fixation

Zusätzlich liefert die Signal-Identifizierungs Aufgabe, die eine komplexe Wahl-Reaktion darstellt und gleichzeitig ein Maß für kognitive Leistungsfähigkeit ist, weitere Parameter, insbesondere die Anzahl der korrekten Reaktionen und die mittlere Reaktionszeit.

Weitere sekundäre Zielparameter wurden mit einem speziellen kognitiven Tests bestimmt, nämlich dem SKT-Tests (Lehfeld und Erzigheit, Int. Psychogeriatr. 1997; 9 Suppl 1: 115-21).

Die Veränderung in den elektrophysiologischen Untersuchungen sowie dem kognitiven Test durch Ketamin wurde jeweils am Ende der einwöchigen Behandlungsperioden durchgeführt. Zunächst erfolgte eine Untersuchung unter Baseline-Bedingungen. Darauf erfolgte über eine Stunde hinweg die Infusion von 4 mg Ketamin über einen Zeitraum von einer Stunde. 30 Minuten nach Beginn der Infusion wurden erneut die AEPs gemessen, der ODT und der SKT-Test durchgeführt.

Die AEP zeigten eine deutliche Reduktion der N1-P2-peak-Amplitude in der Placebo-Bedingung. Diese Verminderung wurde durch Johanniskraut-Extrakt vollständig aufgehoben, so dass sich ein hochsignifikanter Effekt für die Wirksamkeit von Johanniskraut-Extrakt ergab (p=0. 004) (Tab. 1)

**Table 1: 2x2-Cross-Over Analyse**

| **Parameter: Veränderung (Differenz Vor/Nach Ketamine) auf AEP-Parameter (Primare Ziel Variable)** | | | |
|---|---|---|---|
| **Parameter (*µ*V)** | **Medikation** | **Wert** | **Standard Error** |
| **Veränderung der N1/P2-peak-to-peak Amplitude** | Placebo | -8.3946 | 3.8947 |
| | Verum | 10.3174 | 3.8947 |
| **Veränderung der in N1-Amplitude** | Placebo | -4.9315 | 1.8671 |
| | Verum | 3.6559 | 1.8671 |
| **Veränderung der P2-Amplitude** | Placebo | -3.4631 | 2.2432 |
| | Verum | 6.6615 | 2.2432 |

| **Differenz zwischen Plazebo und Verum** | | | |
|---|---|---|---|
| **Parameter (µV)** | **Wert** | **Standard Error** | **p** |
| **Veränderung der N1/P2-peak-to-peak Amplitude** | -18.7121 | 5.5080 | 0.0040 |
| **Veränderung der in N1-Amplitude** | -8.5874 | 2.6405 | 0.0054 |
| **Veränderung der P2-Amplitude** | -10.1246 | 3.1724 | 0.0061 |

Entsprechendes gilt auch für die Einzelkomponenten N1 und P2. Unter Baseline-Bedingungen, d.h. vor der Gabe von Ketamin, zeigte sich kein signifikanter Unterschied zwischen Placebo und Verum. Das zeigt, dass der beschriebenen Effekt als Ketamin-Antagonismus gewertet werden muss.

Im ODT zeigte die Anzahl der korrekten Antworten keinen Unterschied zwischen der Placebo und der Verum-Gruppe. Alle anderen Parameter, nämlich die Auswahl-Reaktionszeit, die Winkelgeschwindigkeit und die Latenz zeigten einen Trend zu einer Beeinträchtigung durch Ketamin und eine ausgeprägte Verminderung der Beeinträchtigung durch Johanniskraut-Extrakt.

Im SKT-Test waren die Beeinträchtigungen durch Ketamin klein und inhomogen, so dass kein Unterschied zwischen Placebo und Verum beobachtet werden konnte.

Die dargestellten Befunde zeigen, dass die Einnahme von 2 x 750 mg Hypericumextrakt einen Ketamin antagonistischen Effekt ausübt. Damit wird deutlich, dass diese Medikation eine therapeutischen Effekt auf die Negativsymptomatik der Schizophrenie haben kann.

Gleiche Effekte lassen sich auch bei der Verwendung von Gingko, Safran und Ginseng beobachten.

## Patentansprüche

1. Verwendung von Hypericum perforatum (Johanniskraut) and/oder Ginkgo biloba (Ginkgo) and/oder Crocus sativus (Safran) and/oder Panax ginseng (Ginseng) zur Herstellung eines Medikaments zur Behandlung von Schizophrenie.

2. Verwendung gemäß Anspruch 1, wobei die Pflanze, Pflanzenteile, getrocknete Pflanze oder Pflanzenteile, Extrakte, Extraktfraktionen, Reinstoffe and deren Derivate and Salze aus der Pflanze verwendet werden.

3. Verwendung gemäß einem der vorherigen Anspruche wobei der verwendete Extrakt ein alkoholischer, alkoholischwässriger Auszug mit primären, sekundären and tertiären Alkoholen der Reihe C1-C5, vorzugsweise Methanol and Ethanol ist, in der Zusammensetzung von Alkohol/Wasser zwischen 100/0 bis 30/70, vorzugsweise 80/20 bis 50/50.

4. Verwendung gemäß Anspruch 3, wobei diese in einem ein and mehrstufigen Herstellverfahren hergestellt sind.

5. Verwendung gemäß einem der Anspruche 1 bis 4, wobei der verwendete Johanniskraut-Extrakt folgende Gehalte an Inhaltsstoffen: 0,01 - 2 % Hypericine, 0,01 - 30.% Hyperforine, 2 - 35 % Flavonoide, vorzugsweise 0,10 - 0,40 % Hypericine and 1 - 6 % Hyperforine aufweist.

6. Verwendung gemäß einem der Anspruche 1 bis 4, wobei der Gingko-Extrakt folgende Gehalte and Inhaltsstoffen: 20 - 30 Gew. % Flavonglycoside, zusammen 2 - 8 Gew. % der Ginkgolide, besonders bevorzugt 23 - 27 % Flavonglycoside and 5 - 7 Gew. % Ginkgolide aufweist.

7. Verwendung gemäß einem der Anspruche 1 bis 4, wobei der Safran-Extrakt die Inhaltsstoffe α-, β-Pinen, 1, 8-Cineol, Crocin, Picrocrocin sowie ggf. dessen Abbauprodukt Safranal, besonders bevorzugt in folgenden Konzentrationen: 5 - 10% Pinene and Cineol, 4 - 10% Picrocrocin and/oder 2 - 6% Safranal aufweist.

8. Verwendung gemäß einem der Anspruche 1 bis 4, wobei der Ginsengwurzel-Extrakt die Inhaltsstoffe u.a. Triterpensaponine (Ginsenoside/Ginsenoide), Sesquiterpene and Polyacetylene, besonders bevorzugt in folgenden Konzentrationen: 3 - 9% Ginsenoide aufweist.

9. Verwendung gemäß einem der vorherigen Anspruche wobei die Verwendung in Form von flüssigen, halbfesten and festen Darreichungsformen, insbesondere Lösungen, Suspensionen, Tabletten, Filmtabletten, Dragees, Kapseln, Brausetabletten, Brausegranulat, Kautabletten, Suppositorien erfolgt.

10. Verwendung gemäß einem der vorherigen Anspruche, wobei die Tagesdosis der Extrakte 300 - 2700 mg in bis zu drei Einzeldosierungen am Tag, vorzugsweise 750 - 1500 mg in 1-2 Einzeldosierungen am Tag, im Falle von Johanniskraut-Extrakt ist.

11. Verwendung gemäß einem der vorherigen Anspruche, wobei die Tagesdosis der Extrakte 50 mg - 1000 mg Extrakt im Falle von Gingko biloba, Crocus sativus and Panax ginseng ist.

12. Kombinationspräparat, enthaltend Johanniskraut, Gingko, Safran and/oder Ginseng sowie als weitere Komponente ein Psychopharmaka gegen Schizophrenie zur gleichzeitigen, getrennten oder zeitlich abgestuften Anwendung bei der Behandlung von Schizophrenie.

13. Verwendung gemäß einem der Anspruche 1 bis 11 in der Add-on Therapie in Kombination mit Psychopharmaka zur Behandlung der Schizophrenie, insbesondere Neuroleptika, insbesondere aus der Gruppe der klassischen Neuroleptika wie Haloperidol, Benperidol, Chlorprotixen, Flupentixol, Fluphenazin, Perazin, Perphenazin, Thioridazin, atypischen Neuroleptika, insbesondere Clozapin, Olanzapin, Seroquel, Sertindol, sowie andere zur Behandlung der Schizophrenie geeignete Psychopharmaka.

## Claims

1. Use of Hypericum perforatum (St. John's wort) and/or Ginkgo biloba (ginkgo) and/or Crocus sativus (saffron) and/or Panax ginseng (ginseng) for the production of a medicament for the treatment of schizophrenia.

2. Use according to claim 1, wherein the plant, plant parts, dried plant or plant parts, extracts, extract fractions, pure materials and derivatives thereof and salts from the plant are used.

3. Use according to one of the previous claims, wherein the extract used is an alcoholic, alcoholic-aqueous extraction using primary, secondary and tertiary alcohols of the series C1-C5, preferably methanol and ethanol, in the composition of alcohol/water between 100/0 to 30/70, preferably 80/20 to 50/50.

4. Use according to claim 3, wherein they are produced in a single-stage and multi-stage production process.

5. Use according to one of claims 1 to 4, wherein the St. John's wort extract used has the following levels of components: 0.01 - 2 % hypericins, 0.01 - 30 % hyperforins, 2 - 35 % flavonoids, preferably 0.10 - 0.40 % hypericins and 1-6 % hyperforins.

6. Use according to one of claims 1 to 4, wherein the gingko extract has the following levels and components: 20 -30 wt.% flavonglycosides, together 2 - 8 wt.% of ginkgolides, particularly preferably 23 - 27 % flavonglycosides and 5 - 7 wt.% ginkgolides.

7. Use according to one of claims 1 to 4, wherein the saffron extract has the components α-pinene, β-pinene, 1,8-cineole, crocin, picrocrocin and optionally its degradation product safranal, particularly preferably in the following concentrations: 5 - 10 % pinenes and cineole, 4 - 10 % picrocrocin and/or 2 - 6 % safranal.

8. Use according to one of claims 1 to 4, wherein the ginseng root extract has the components, inter alia, triterpene saponins (ginsenosides/ginsenoids), sesquiterpenes and polyacetylene, particularly preferably in the following concentrations: 3 - 9 % ginsenoids.

9. Use according to one of the previous claims, wherein the use is effected in the form of liquid, semi-solid and solid forms of administration, in particular solutions, suspensions, tablets, film tablets, coated tablets, capsules, effervescent tablets, effervescent granules, lozenges, suppositories.

10. Use according to one of the previous claims, wherein the daily dose of the extracts is 300 - 2,700 mg in up to three single doses during the day, preferably 750 -1,500 mg in 1-2 single doses during the day, in the case of St. John's wort extract.

11. Use according to one of the previous claims, wherein the daily dose of the extracts is 50 mg -1,000 mg of extract in the case of Gingko biloba, Crocus sativus and Panax ginseng.

12. Combination preparation containing St. John's wort, gingko, saffron and/or ginseng and as a further component a psychopharmaceutical for schizophrenia for the simultaneous, separate or temporally gradual administration in the treatment of schizophrenia.

13. Use according to one of claims 1 to 11 in add-on therapy in combination with psychopharmaceuticals for the treatment of schizophrenia, in particular neuroleptics, in particular from the group of classical neuroleptics, such as haloperidol, benperidol, chlorprotixen, flupentixol, fluphenazine, perazine, perphenazine, thioridazine, atypical neuroleptics, in particular clozapine, olanzapine, seroquel, sertindole, and other psychopharmaceuticals suitable for the treatment of schizophrenia.

## Revendications

1. Utilisation de l'hypericum perforatum (millepertuis ou herbe de la saint jean) et/ou de ginkgo biloba (ginkgo) et/ou de crocus sativus (safran) et/ou de panax ginseng (ginseng), pour la préparation d'un médicament pour le traitement de la schizophrénie.

2. Utilisation selon la revendication 1, où la plante, les parties de plante, la plante ou des parties de plante séchée(s), des extraits, des fractions d'extrait, des substances pures et leurs dérivés et sels issus de la plante, sont utilisés.

3. Utilisation selon l'une des revendications précédentes, sachant que l'extrait utilisé est un extrait alcoolique, hydro-alcoolique avec des alcools primaires, secondaires et tertiaires de la série en C1 à C5, de préférence le méthanol et l'éthanol dans la composition alcool/eau dans la fourchette entre 100/0 et 30/70, de préférence de 80/20 à 50/50.

4. Utilisation selon la revendication 3, celles-ci étant préparées dans un procédé de préparation à une et plusieurs étapes.

5. Utilisation selon l'une des revendications 1 à 4, l'extrait de millepertuis utilisé présentant les teneurs suivantes en composantes : de 0,01 à 2 % d'hypéricine, de 0,01 à 30 % d'hyperforine, de 2 à 35% de flavonoïde, et, de préférence, de 0,10 à 0,40 % d'hypéricine et de 1 à 6 % d'hyperforine.

6. Utilisation selon l'une des revendications 1 à 4, l'extrait de gingko présentant les teneurs et composants suivants : de 20 à 30 % en poids de flavonglycoside, ensemble de 2 à 8 % en poids des ginkgolides, de façon particulièrement préférée, 23 à 27 % en poids de flavonglycoside, et 5 à 7 % en poids de ginkgolides.

7. Utilisation selon l'une des revendications 1 à 4, l'extrait de safran présentant les contenus α-, β-pinène, 1,8-cineol, crocine, picrocrocine, ainsi que, le cas échéant, son produit de décomposition, le safranal, de façon particulièrement préférée sous les concentrations suivantes : de 5 à 10 % de pinène et cinéole, de 4 à 10 % de picrocrocine et/ou 2 à 6 % de safranal.

8. Utilisation selon l'une des revendications 1 à 4, l'extrait de racine de ginseng présentant les composants entre autres : triterpensaponine (ginsénoside/ginsénoïde), sesquiterpène et polyacétyléne, de façon particulièrement préférée sous les concentrations suivantes : 3 à 9 % de ginsénoïde.

9. Utilisation selon l'une des revendications précédentes, sachant que l'utilisation se fait sous la forme de formes d'administration liquides, semi-solides et solides, en particulier de solutions, de suspensions, de cachets, de cachets en film, de dragées, de capsules, de comprimés effervescents, de granulés effervescents, de comprimés à mâcher, de suppositoires.

10. Utilisation selon l'une des revendications précédentes, la dose quotidienne des extraits étant de 300 à 2700 mg jusqu'à trois dosages individuels quotidiens, de préférence de 750 à 1500 mg en 1 à 2 dosages individuels quotidiens, dans le cas de l'extrait de millepertuis.

11. Utilisation selon l'une des revendications précédentes, la dose quotidienne des extraits étant de 50 mg à 1000 mg d'extrait dans le cas de gingko biloba, crocus sativus et panax ginseng.

12. Préparation de combinaisons contenant du millepertuis, du gingko, du safran et/ou du ginseng, ainsi que, en tant qu'autres composants, un produit de la psychopharmacopée contre la schizophrénie, pour application simultanée, séparée ou temporellement échelonnée, lors du traitement de la schizophrénie.

13. Utilisation selon l'une des revendications 1 à 11 dans la thérapie add-on, en combinaison avec un produit de la psychopharmacopée pour le traitement de la schizophrénie, en particulier d'un neuroleptique, en particulier du groupes des neuroleptiques classiques, tels que halopéridol, benpéridol, chlorprotixène, flupentixol, fluphénazine, pérazine, perphénazine, thioridazine, neuroleptiques atypiques, en particulier clozapine, olanzapine, seroquèle, sertindol, ainsi que d'autres produits de psychopharmacopée, convenant pour le traitement de la schizophrénie.
